# FASCICULE DE BREVET EUROPEEN

(11) **EP 1 865 932 B1**
(45) Date de publication et mention de la délivrance du brevet: **03.12.2008**
(21) Numéro de dépôt: 06726013.3
(22) Date de dépôt: 03.03.2006
(51) Int. Cl.: A61K 9/70

(54) **TIMBRE DERMIQUE RECHARGEABLE**
AUFLADBARES HAUTPFLASTER
RECHARGEABLE DERMAL PATCH

(30) Priorité: 08.04.2005 FR 0503499
(43) Date de publication de la demande: 19.12.2007
(73) Titulaire: Millet Innovation, 26270 Loriol sur Drome (FR)
(72) Inventeur: MILLET, Jean-Claude, F-26800 Etoile sur Rhône (FR); MARTIN, Jean-Luc, F-26270 Loriol sur Drôme (FR)
(74) Mandataire: Marchand, André
(86) Numéro de dépôt international: PCT/FR2006/000474
(87) Numéro de publication internationale: WO 2006/108933

(56) Documents cités:
- EP-A- 0 412 869
- EP-A- 0 976 383
- FR-A- 2 113 778
- US-A- 4 913 905

## Description

La présente invention concerne un timbre dermique à usage médical ou cosmétique, assurant la diffusion plus ou moins lente vers la peau d'un principe actif.

Il est connu d'appliquer sur la peau des films permettant d'apporter à la peau, et le cas échéant, de faire pénétrer progressivement dans la peau par transdermie, des produits pharmaceutiques (allopathiques et/ou homéopathiques et/ou de phytothérapie), et/ou cosmétiques, notamment des solutions aqueuses.

On a ainsi déjà proposé des films composites comportant plusieurs couches successives dont une couche dite "couche réservoir", généralement mise en contact avec la peau et contenant le principe actif constitué d'un ou plusieurs produits traitants.

Dans le brevet EP 0 412 869, cette couche réservoir est constituée d'une matrice polymère de silicone pouvant contenir jusqu'à 50 % en poids de phase aqueuse gélifiée par rapport au poids total de la couche réservoir, le principe actif étant mélangé à la phase aqueuse gélifiée. Le polymère n'est que partiellement polymérisé et présente un caractère auto-adhésif. Une trame incluse dans cette couche réservoir permet de la renforcer mécaniquement.

La couche réservoir peut être préparée en mélangeant sous agitation la phase aqueuse active déjà gélifiée dans un polymère de silicone non polymérisé. Elle peut être également préparée en mélangeant sous agitation un polymère de silicone non polymérisé, la phase aqueuse active et l'agent gélifiant.

Il s'avère que ce procédé est limitatif en terme de dosage du principe actif puisque celui-ci ne peut pas excéder 0,5 à 1 %, et exceptionnellement jusqu'à 5% en poids de la couche réservoir. Au-delà de cette valeur, la polymérisation permettant d'obtenir une structure matricielle ne se produit pas. On a en outre observé que certains principes actifs même en très faible proportion bloquent la polymérisation.

De plus, comme le principe actif est incorporé à la couche réservoir lors de la fabrication de celle-ci, ce procédé nécessite la fabrication d'un film distinct pour chaque principe actif et pour chaque dosage souhaité du principe actif, ce qui complique la fabrication et la gestion des stocks des timbres dermiques. Par ailleurs, une fois que le principe actif dans la couche réservoir a été complètement absorbé par la peau de l'utilisateur, un timbre n'est pas réutilisable sans recyclage, ce qui augmente son coût d'utilisation.

La présente invention a pour but de remédier à ces inconvénients en proposant un timbre dermique dans lequel la proportion en poids du principe actif n'est pas limitée par les contraintes de polymérisation, et dans lequel on peut réintroduire un principe actif plusieurs fois lorsque la quantité de principe actif précédemment introduite dans le timbre a été absorbée par la peau de l'utilisateur, le principe actif réintroduit pouvant être identique ou différent de celui précédemment introduit.

A cet effet, la présente invention prévoit un timbre dermique comprenant, en sus d'une couche réservoir, une couche réceptrice pour recevoir un liquide contenant un principe actif et le transmettre à la couche réservoir.

Plus particulièrement, la présente invention prévoit un timbre dermique comprenant une couche réservoir en un gel polymère prévue pour recevoir un liquide contenant un principe actif et présentant une première face à appliquer sur la peau d'un utilisateur en vue de diffuser le principe actif.

Selon l'invention, le timbre dermique comprend en outre :
- une couche réceptrice pour recevoir le liquide contenant le principe actif et transmettre le liquide à la couche réservoir, et
- une couche de colle assurant le maintien de la couche réceptrice sur une seconde face de la couche réservoir et ne s'opposant pas au transfert de liquide de la couche réceptrice vers la couche réservoir.

Selon un mode de réalisation de l'invention, la couche réservoir comprend un polyméthylsiloxane résultat de la polymérisation d'un mélange d'huiles de silicone.

Selon un mode de réalisation de l'invention, la couche réservoir comprend du dimethicone ou un mélange de dimethicone et de vinyl dimethicone.

Selon un mode de réalisation de l'invention, la couche réceptrice est en un tissu qui est élastique au moins dans une direction.

Selon un mode de réalisation de l'invention, la couche réceptrice est en un tissu polyamide élasthanne de type tissé.

Selon un mode de réalisation de l'invention, la couche réceptrice présente un grammage de l'ordre de la centaine ou quelques centaines de grammes au mètre carré et une épaisseur inférieure au millimètre.

Selon un mode de réalisation de l'invention, la couche réservoir présente une épaisseur comprise entre 0,2 et 2 mm.

Selon un mode de réalisation de l'invention, la première face de la couche réservoir est auto-adhésive.

Selon un mode de réalisation de l'invention, la couche de colle est d'une faible épaisseur de l'ordre du dixième à quelques dixièmes de millimètres, pour ne pas empêcher le transfert du liquide comprenant le principe actif entre la couche réceptrice et la couche réservoir, et pénétrer le moins possible dans la couche réceptrice.

Selon un mode de réalisation de l'invention, la couche de colle est une colle acétique.

L'invention concerne également un procédé de fabrication d'un timbre dermique comprenant une étape de fabrication d'une couche réservoir par polymérisation d'un gel polymère, la couche réservoir étant prévue pour recevoir un liquide contenant un principe actif et diffuser le liquide dans la peau de l'utilisateur.

Selon l'invention, le procédé comprend en outre les étapes suivantes :
- préparation d'une couche réceptrice,
- assemblage de la couche réceptrice et de la couche réservoir au moyen d'une couche de colle,

la couche réceptrice étant réalisée en un matériau capable de recevoir le liquide contenant le principe actif et le transmettre à la couche réservoir, la couche de colle étant réalisée de manière à ce qu'elle ne s'oppose pas au transfert de liquide de la couche réceptrice vers la couche réservoir.

Selon un mode de réalisation de l'invention, le procédé comprend une étape de versement d'une dose d'un liquide contenant un principe actif sur la couche réceptrice.

Selon un mode de réalisation de l'invention, un liquide contenant un principe actif est mélangé au gel de silicone préalablement à la polymérisation de celui-ci.

Selon un mode de réalisation de l'invention, la couche réceptrice et la couche réservoir sont assemblées au moyen d'une couche de colle de faible épaisseur de l'ordre du dixième à quelques dixièmes de millimètres, pour ne pas empêcher le transfert du liquide comprenant le principe actif de la couche réceptrice vers la couche réservoir, et pénétrer le moins possible dans la couche réceptrice.

Ces objets, caractéristiques et avantages ainsi que d'autres de la présente invention seront exposés plus en détail dans la description suivante de l'invention faite à titre non limitatif en relation avec les figures jointes parmi lesquelles :
- la figure 1 est une vue en perspective d'un timbre dermique selon l'invention, et
- la figure 2 est une vue en coupe transversale du timbre dermique.

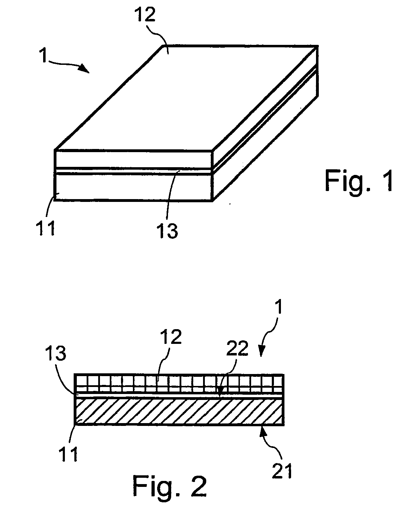

Les figures 1 et 2 représentent un timbre dermique 1 comprenant une couche réservoir 11 prévue pour retenir un principe actif et le diffuser au travers de la peau d'un utilisateur, la couche 11 présentant une face 21 destinée à être appliquée sur la peau de l'utilisateur. Selon l'invention, le timbre dermique comprend en outre une couche réceptrice 12 extérieure prévue pour recevoir un liquide contenant le principe actif et pour le transmettre à la couche réservoir 11 par migration de celui-ci. La couche réservoir 11 et la couche réceptrice 12 sont assemblées au moyen d'une couche de colle 13 qui est réalisée de manière à ne pas empêcher la transmission du liquide contenant le principe actif, de la couche réceptrice 12 à la couche réservoir 11.

Dans un exemple de réalisation de l'invention, la couche réservoir 11 est réalisée à partir d'un mélange polymérisé de deux ou trois huiles de silicone, qui présente l'avantage d'être auto-adhésif sur la peau de l'utilisateur. Ce caractère auto-adhésif, en soi classique, s'explique par le fait que le processus de polymérisation des huiles de silicone mélangées n'est généralement pas complet, de sorte que le matériau présente après polymérisation une structure matricielle contenant des huiles de silicone non polymérisées.

Le gel obtenu présente par exemple une structure matricielle en polyméthylsiloxane et notamment en polydiméthylsiloxane qui retient les huiles de silicone non polymérisées, lesquelles comportent du dimethicone ou un mélange de dimethicone et de vinyl dimethicone. L'épaisseur de la couche réservoir 11 est par exemple comprise entre 0,2 et 2 mm.

La couche réceptrice 12 peut être réalisée en un tissu qui est avantageusement élastique au moins dans une direction, et de préférence suivant deux directions distinctes pour permettre au timbre de s'adapter à la forme de la région de la peau de l'utilisateur où il est appliqué. Ce tissu est par exemple du tissu polyamide élasthanne de type tissé (comprenant des mailles de tissage) comprenant par exemple 80 % de polyamide et 20 % d'élasthanne, et un grammage de l'ordre de 180 g/m². Dans ces conditions, l'épaisseur de la couche réceptrice est par exemple de l'ordre de 0,6 mm.

La colle d'assemblage 13 de la couche réservoir 11 et de la couche réceptrice 12 est de préférence une colle acétique, par exemple une colle silicone à l'acide acétique. L'épaisseur de la couche de colle est de préférence faible, par exemple de l'ordre de 0,1 à 0,2 mm, pour ne pas s'opposer à la migration du liquide comprenant le principe actif entre la couche réceptrice et la couche réservoir, et pour pénétrer le plus faiblement possible entre les mailles du tissu formant la couche réceptrice, afin de ne pas réduire la capacité d'absorption de liquide de cette dernière.

Le principe actif introduit dans le timbre comprend une composition pharmaceutique (allopathique et/ou homéopathique et/ou de phytothérapie) et/ou cosmétique, telle qu'une ou plusieurs huiles essentielles ayant des vertus thérapeutiques et/ou cosmétiques.

Le timbre dermique selon l'invention est par exemple fabriqué comme indiqué ci-après.

La couche réservoir 11 est tout d'abord réalisée à partir d'un mélange d'huiles de silicone, pour obtenir un gel de silicone tel le PDMS. La structure obtenue est une structure matricielle qui retient les huiles de silicone non polymérisées. Le principe actif n'est pas mélangé aux huiles de silicone avant la polymérisation, de sorte que l'on s'affranchit des problèmes de blocage de polymérisation qui peuvent être provoqués par le liquide contenant le principe actif, si celui-ci est incompatible, totalement ou partiellement, à dosage élevé. Toutefois, si le liquide contenant le principe actif est compatible avec la polymérisation, il peut être mélangé avec les huiles de silicone avant leur polymérisation.

Une face 22 de la couche réservoir 11, opposée à la face 21, est ensuite enduite de colle 13. Puis, la couche réceptrice 12 est appliquée sur la face 22, afin que les couches 11, 12 se trouvent assemblées après séchage de la colle 13. Comme indiqué plus haut, la colle 13 est employée dans une quantité telle qu'elle ne traverse pas les mailles du tissu à l'issue de l'application des deux couches l'une contre l'autre.

Dans une variante de réalisation, la colle est déposée sur la couche réceptrice 12 et non sur la couche réservoir 11. La colle peut également être déposée à la fois sur couche réceptrice 12 et la couche réservoir 11, sous réserve de l'épaisseur finale de colle obtenue et à la condition que la colle ne s'oppose pas ensuite à la migration du liquide.

Enfin, la face 21 de la couche réservoir 11 est de préférence protégée par un film anti-adhésif, tel qu'un film de polyéthylène, destiné à être enlevé avant d'appliquer le timbre sur la peau.

La forme et les dimensions du timbre dermique 1 selon l'invention sont adaptées à la forme et à l'étendue de la zone du corps de l'utilisateur sur laquelle le timbre doit être appliqué. D'une manière générale, le timbre présente une forme carrée ou rectangulaire, d'une longueur et d'une largeur de quelques centimètres à une dizaine de centimètres. Le timbre peut également présenter la forme d'une équerre, par exemple lorsqu'il est destiné à être appliqué sur l'épaule. En outre, il peut être découpé aux dimensions souhaitées au moment de son application sur le corps de l'utilisateur.

Le timbre dermique selon l'invention peut également être obtenu en fabriquant un film composite de grandes dimensions par assemblage d'une couche réceptrice et d'une couche réservoir, puis en découpant le film pour obtenir des timbres aux dimensions souhaitées. La couche réceptrice peut ainsi être distribuée en rouleau au-dessus de la couche réservoir, l'une et/ou l'autre de ces deux couches étant préalablement enduite de colle.

Le liquide contenant le principe actif choisi est appliqué sur la face apparente de la couche réceptrice 12 par un moyen permettant de doser la quantité de liquide apportée, par exemple une pipette ou un emballage unidose. La quantité de liquide transférée dans le timbre dépend de deux paramètres, à savoir la quantité de liquide que la couche réceptrice 12 est capable d'absorber en une fois, et la quantité de principe actif que l'on souhaite transférer vers la peau de l'utilisateur.

On observe que le liquide appliqué sur le timbre se répartit presque instantanément dans toute la couche réceptrice 12, qui sert ainsi de réservoir intermédiaire où le liquide est tout d'abord retenu. Dans l'exemple de réalisation décrit ci-dessus, le liquide est ainsi absorbé par la trame du tissu qui n'a pas été remplie par la colle sous l'effet de la tension superficielle relative entre le liquide et les fils du tissu. Le transfert de liquide de la couche réceptrice 12 vers le réservoir 11 s'effectue ainsi sur toute leur interface. On constate ainsi que le liquide migre très rapidement vers la couche réservoir 11, puis au travers de la couche réservoir vers la face auto-adhésive 21 destinée à être mise en contact avec la peau de l'utilisateur. La couche réceptrice 12 a donc tendance à sécher rapidement.

Dans la couche réservoir 11, le liquide contenant le principe actif se mélange avec les huiles de silicone non polymérisées qui sont réparties dans toute la couche réservoir et retenues par la matrice polymérisée.

La quantité de principe actif susceptible d'être apportée est importante puisque celui-ci ne risque pas d'empêcher la formation par polymérisation partielle du gel polymère constituant la couche réservoir 11. Toutefois, le liquide contenant le principe actif est de préférence miscible avec les huiles silicones. Il convient donc d'utiliser de préférence un liquide hydrophobe et apolaire. À défaut de pouvoir dissoudre naturellement le principe actif dans un liquide hydrophobe, on peut réaliser à partir du principe actif des micro- ou nano-émulsions, ou faire appel à des molécules support telles que des cyclodextrines pour enfermer le principe actif dans un mélange hydrophobe.

Plus la quantité de principe actif introduite dans la couche réservoir est importante plus la vitesse de transfert du principe actif vers la peau de l'utilisateur est importante. Si on utilise certaines huiles essentielles, la diffusion sur la peau ou toute surface de réception compatible commence quelques dizaines de secondes après l'application du timbre. Pour certaines pathologies, on observe un effet sur l'utilisateur une dizaine de minutes seulement après l'application du timbre. On observe également que le timbre continue à diffuser le principe actif après une dizaine de jours d'application continue.

La nature du liquide porteur de principe actif peut bien entendu également influencer les performances du timbre dermique, en termes de vitesse d'absorption du liquide par la couche réceptrice 12 et de vitesse de migration de celui-ci dans la couche réservoir 11. Le liquide utilisé doit notamment être compatible avec le matériau formant la couche réservoir, comme cela est en soi bien connu de l'homme de l'art, pour être bien absorbé par celle-ci.

Un timbre dermique selon l'invention peut recevoir, simultanément ou successivement, plusieurs liquides différents contenant des principes actifs complémentaires dans des proportions prédéfinies.

On peut ainsi par exemple appliquer simultanément ou successivement un ou plusieurs anti-inflammatoires et une composition destinée à faciliter la pénétration des liquides dans la peau. Le timbre peut ainsi être rechargé plusieurs fois pour un traitement prolongé soit avec le même liquide, soit avec des liquides contenant des principes actifs différents pour des traitements complémentaires.

Puisque la vitesse de diffusion vers la peau d'un principe actif inséré dans le timbre dermique selon l'invention dépend notamment de la concentration du principe actif dans la couche réservoir, on peut introduire deux principes actifs dans la couche réservoir à des dosages différents, de manière à combiner l'effet du principe actif introduit en faible quantité qui donc se diffuse lentement, avec le principe apporté en quantité plus importante qui se diffuse plus rapidement.

Il apparaîtra clairement à l'homme de l'art que le timbre dermique selon l'invention est susceptible de diverses variantes de réalisation en ce qui concerne notamment chacun de ses matériaux constitutifs.

Ainsi, l'invention n'exclut pas les compositions non auto-adhésives ou insuffisamment adhésives pour assurer le maintien du timbre sur la peau. Dans un tel cas, le timbre peut être agencé sur un matériau autocollant de type sparadrap, présentant sur sa face arrière une fenêtre permettant l'introduction du liquide sur la couche réceptrice 12.

Par ailleurs, la couche réservoir 11 peut comprendre plusieurs couches de gel polymère assemblées sans adhésif, par simple contact mutuel.

Egalement, la couche réceptrice 12 peut être formée d'un tissu non tissé, et de façon générale tout matériau assurant de façon satisfaisante la fonction de réception du liquide et de transfert de celui-ci dans la couche réservoir 11. Ainsi, s'il est souhaité utiliser un autre matériau qu'un tissu tissé pour former la couche réceptrice, il convient de s'assurer que le liquide se répand rapidement sur toute la surface du matériau et pas seulement de façon locale, afin que la surface de migration du liquide dans la couche réservoir 11 soit maximale. Si les tissus tissés en raison de leur maillage présentent cette propriété d'étalement rapide de toute goutte de liquide qui leur est appliquée, certains tissus non tissés ou matériaux poreux sont susceptibles de présenter également cet avantage mais devront faire, à titre de vérification, l'objet d'essais à la portée de l'homme de l'art.

## Revendications

1. Timbre dermique comprenant une couche réservoir (11) en un gel polymère prévue pour recevoir un liquide contenant au moins un principe actif et présentant une première face (21) à appliquer sur la peau d'un utilisateur en vue de diffuser le principe actif,
**caractérisé en ce qu'**il comprend en outre :
- une couche réceptrice (12) pour recevoir le liquide contenant le principe actif et transmettre le liquide à la couche réservoir, et
- une couche de colle (13) assurant le maintien de la couche réceptrice (12) sur une seconde face (22) de la couche réservoir (11) et ne s'opposant pas au transfert de liquide de la couche réceptrice (12) vers la couche réservoir (11).

2. Timbre selon la revendication 1, dans lequel la couche réservoir (11) comprend un polyméthylsiloxane résultat de la polymérisation d'un mélange d'huiles de silicone.

3. Timbre selon l'une des revendications 1 et 2, dans lequel la couche réservoir (11) comprend du dimethicone ou un mélange de dimethicone et de vinyl dimethicone.

4. Timbre selon l'une des revendications 1 à 3, dans lequel la couche réceptrice (12) est en un tissu qui est élastique au moins dans une direction.

5. Timbre selon l'une des revendications 1 à 4, dans lequel la couche réceptrice (12) est en un tissu polyamide élasthanne de type tissé.

6. Timbre selon l'une des revendications 1 à 5, dans lequel la couche réceptrice (12) présente un grammage de l'ordre de la centaine ou quelques centaines de grammes au mètre carré et une épaisseur inférieure au millimètre.

7. Timbre selon l'une des revendications 1 à 6, dans lequel la couche réservoir (11) présente une épaisseur comprise entre 0,2 et 2 mm.

8. Timbre selon l'une des revendications 1 à 7, dans lequel la première face (21) de la couche réservoir (11) est auto-adhésive.

9. Timbre selon l'une des revendications 1 à 8, dans lequel la couche de colle (13) est d'une faible épaisseur de l'ordre du dixième à quelques dixièmes de millimètres, pour ne pas empêcher le transfert du liquide comprenant le principe actif entre la couche réceptrice (12) et la couche réservoir (11), et pénétrer le moins possible dans la couche réceptrice (12).

10. Timbre selon l'une des revendications 1 à 9, dans lequel la couche de colle (13) est une colle acétique.

11. Procédé de fabrication d'un timbre dermique comprenant une étape de fabrication d'une couche réservoir (11) par polymérisation d'un gel polymère, la couche réservoir étant prévue pour recevoir un liquide contenant un principe actif et diffuser le liquide vers la peau de l'utilisateur,
**caractérisé en ce qu'**il comprend en outre les étapes suivantes :
- préparation d'une couche réceptrice (12),
- assemblage de la couche réceptrice (12) et de la couche réservoir (11) au moyen d'une couche de colle (13),
la couche réceptrice (12) étant réalisée en un matériau capable de recevoir le liquide contenant le principe actif et le transmettre à la couche réservoir (11), la couche de colle (13) étant réalisée de manière à ce qu'elle ne s'oppose pas au transfert de liquide de la couche réceptrice (12) vers la couche réservoir (11).

12. Procédé selon la revendication 11, comprenant une étape de versement d'une dose d'un liquide contenant un principe actif sur la couche réceptrice (12).

13. Procédé selon la revendication 11 ou 12, dans lequel un liquide contenant un principe actif est mélangé au gel de silicone préalablement à la polymérisation de celui-ci.

14. Procédé selon l'une des revendications 11 à 13, dans lequel la couche réceptrice (12) est en un tissu qui est élastique au moins dans une direction.

15. Procédé selon l'une des revendications 11 à 14, dans lequel la couche réceptrice (12) est en un tissu polyamide élasthanne de type tissé.

16. Procédé selon l'une des revendications 11 à 15, dans lequel la couche réceptrice (12) présente un grammage de la centaine ou quelques centaines de grammes au mètre carré et une épaisseur inférieure au millimètre.

17. Procédé selon l'une des revendications 11 à 16, dans lequel la couche réceptrice (12) et la couche réservoir (11) sont assemblées au moyen d'une couche de colle (13) de faible épaisseur de l'ordre du dixième à quelques dixièmes de millimètres, pour ne pas empêcher le transfert du liquide comprenant le principe actif de la couche réceptrice (12) vers la couche réservoir (11), et pénétrer le moins possible dans la couche réceptrice (12).

18. Procédé selon l'une des revendications 11 à 17, dans lequel la couche réservoir (11) comprend un polyméthylsiloxane résultat de la polymérisation d'un mélange d'huiles de silicone.

19. Procédé selon l'une des revendications 11 à 18, dans lequel la couche réservoir (11) comprend du dimethicone ou un mélange de dimethicone et de vinyl dimethicone.

20. Procédé selon l'une des revendications 11 à 19, dans lequel la première face (21) de la couche réservoir (11) est auto-adhésive.

21. Procédé selon l'une des revendications 11 à 20,
dans lequel la couche de colle (13) est une colle acétique.

## Claims

1. A dermal patch comprising a reservoir layer (11) made of a polymer gel provided for receiving a liquid containing at least one active substance and having a first face (21) to be applied onto the skin of a user in order to diffuse the active substance,
**characterized in that** it further comprises:
- a receiving layer (12) for receiving the liquid containing the active substance and transmitting the liquid to the reservoir layer, and
- a layer of glue (13) in charge of holding the receiving layer (12) on a second face (22) of the reservoir layer (11) and which does not hinder the transfer of liquid from the receiving layer (12) to the reservoir layer (11).

2. Patch according to claim 1, wherein the reservoir layer (11) comprises a polymethylsiloxane resulting from the polymerization of a mixture of silicone oils.

3. Patch according to one of claims 1 and 2, wherein the reservoir layer (11) comprises dimethicone or a mixture of dimethicone and vinyl dimethicone.

4. Patch according to one of claims 1 to 3, wherein the receiving layer (12) is made of a fabric which is elastic at least in one direction.

5. Patch according to one of claims 1 to 4, wherein the receiving layer (12) is made of a woven-type elastane polyamide fabric.

6. Patch according to one of claims 1 to 5, wherein the receiving layer (12) has a basis weight in the order of one hundred or a few hundred grams per square meter and a thickness lower than one millimeter.

7. Patch according to one of claims 1 to 6, wherein the reservoir layer (11) has a thickness between 0.2 and 2 mm.

8. Patch according to one of claims 1 to 7, wherein the first face (21) of the reservoir layer (11) is self-adhesive.

9. Patch according to one of claims 1 to 8, wherein the layer of glue (13) is thin, in the order of one tenth to a few tenths of a millimeter, so as not to prevent the transfer of the liquid comprising the active substance between the receiving layer (12) and the reservoir layer (11), and to penetrate as little as possible into the receiving layer (12).

10. Patch according to one of claims 1 to 9, wherein the layer of glue (13) is an acetic glue.

11. A method for manufacturing a dermal patch comprising a step of manufacturing a reservoir layer (11) by polymerizing a polymer gel, the reservoir layer being provided for receiving a liquid containing an active substance and diffusing the liquid towards the user's skin,
**characterized in that** it further comprises the following steps of:
- preparing a receiving layer (12),
- assembling the receiving layer (12) and the reservoir layer (11) by means of a layer of glue (13),
the receiving layer (12) being produced in a material capable of receiving the liquid containing the active substance and transmitting it to the reservoir layer (11), the layer of glue (13) being produced so that it does not hinder the transfer of liquid from the receiving layer (12) towards the reservoir layer (11).

12. Method according to claim 11, comprising a step of pouring a dose of a liquid containing an active substance onto the receiving layer (12).

13. Method according to claim 11 or 12, wherein a liquid containing an active substance is mixed with the silicone gel prior to the polymerization of the latter.

14. Method according to one of claims 11 to 13, wherein the receiving layer (12) is made of a fabric which is elastic at least in one direction.

15. Method according to one of claims 11 to 14, wherein the receiving layer (12) is made of a woven-type elastane polyamide fabric.

16. Method according to one of claims 11 to 15, wherein the receiving layer (12) has a basis weight of one hundred or a few hundred grams per square meter and a thickness lower than one millimeter.

17. Method according to one of claims 11 to 16, wherein the receiving layer (12) and the reservoir layer (11) are assembled by means of a thin layer of glue (13) in the order of one tenth to a few tenths of a millimeter, so as not to prevent the transfer of the liquid comprising the active substance from the receiving layer (12) to the reservoir layer (11), and to penetrate as little as possible into the receiving layer (12).

18. Method according to one of claims 11 to 17, wherein the reservoir layer (11) comprises a polymethylsiloxane resulting from the polymerization of a mixture of silicone oils.

19. Method according to one of claims 11 to 18, wherein the reservoir layer (11) comprises dimethicone or a mixture of dimethicone and vinyl dimethicone.

20. Method according to one of claims 11 to 19, wherein the first face (21) of the reservoir layer (11) is self-adhesive.

21. Method according to one of claims 11 to 20, wherein the layer of glue (13) is an acetic glue.

## Patentansprüche

1. Hautpflaster, umfassend eine Speicherschicht (11) aus einem Polymergel, die dazu vorgesehen ist, eine Flüssigkeit aufzunehmen, die mindestens ein Aktivprinzip enthält, und die eine erste Seite (21) aufweist, die auf die Haut eines Benutzers aufzubringen ist, um das Aktivprinzip zu verteilen,
**dadurch gekennzeichnet, dass** es ferner umfasst:
- eine Aufnahmeschicht (12) für die Aufnahme der Flüssigkeit, die das Aktivprinzip enthält, und für die Weiterleitung der Flüssigkeit an die Speicherschicht, und
- eine Klebeschicht (13), die die Haftung der Aufnahmeschicht (12) auf einer zweiten Seite (22) der Speicherschicht (11) sichert und für die Weiterleitung der Flüssigkeit von der Aufnahmeschicht (12) zur Speicherschicht (11) nicht hinderlich ist.

2. Pflaster nach Anspruch 1, bei dem die Speicherschicht (11) ein Polymethylsiloxan umfasst, das aus der Polymerisierung eines Silikonölgemisches stammt.

3. Pflaster nach einem der Ansprüche 1 und 2, bei dem die Speicherschicht (11) Dimethicon oder ein Gemisch aus Dimethicon und Vinyldimethicon umfasst.

4. Pflaster nach einem der Ansprüche 1 bis 3, bei dem die Aufnahmeschicht (12) aus einem Gewebe besteht, das mindestens in eine Richtung elastisch ist.

5. Pflaster nach einem der Ansprüche 1 bis 4, bei dem die Aufnahmeschicht (12) aus einem Elasthanpolyamidgewebe gewebten Typs ist.

6. Pflaster nach einem der Ansprüche 1 bis 5, bei dem die Aufnahmeschicht (12) eine Flächenmasse von ungefähr einem Hundertstel oder einigen Hundertstel Gramm pro Quadratmeter und eine Dicke unter einem Millimeter aufweist.

7. Pflaster nach einem der Ansprüche 1 bis 6, bei dem die Speicherschicht (11) eine Dicke zwischen 0,2 und 2 mm aufweist.

8. Pflaster nach einem der Ansprüche 1 bis 7, bei dem die erste Seite (21) der Speicherschicht (11) selbst haftend ist.

9. Pflaster nach einem der Ansprüche 1 bis 8, bei dem die Klebeschicht (13) von geringer Dicke von ungefähr einem Zehntel bis zu einigen Zehntel Millimeter ist, um die Weiterleitung der Flüssigkeit, die das Aktivprinzip enthält, zwischen der Aufnahmeschicht (12) und der Speicherschicht (11) nicht zu behindern und möglichst wenig in die Aufnahmeschicht (12) einzudringen.

10. Pflaster nach einem der Ansprüche 1 bis 9, bei dem die Klebeschicht (13) ein Essigkleber ist.

11. Verfahren zur Herstellung eines Hautpflasters, umfassend einen Schritt der Herstellung einer Speicherschicht (11) durch Polymerisierung eines Polymergels, wobei die Speicherschicht dazu vorgesehen ist, eine Flüssigkeit, die ein Aktivprinzip enthält, aufzunehmen und die Flüssigkeit auf der Haut des Benutzers zu verteilen,
**dadurch gekennzeichnet, dass** es ferner die folgenden Schritte umfasst:
- Herstellung einer Aufnahmeschicht (12),
- Verbindung der Aufnahmeschicht (12) und der Speicherschicht (11) mittels einer Klebeschicht (13),
wobei die Aufnahmeschicht (12) aus einem Material hergestellt ist, das die Flüssigkeit, die das Aktivprinzip enthält, aufnehmen und an die Speicherschicht (11) weiterleiten kann, wobei die Klebeschicht (13) derart ausgeführt ist, dass sie die Weiterleitung der Flüssigkeit von der Aufnahmeschicht (12) zur Speicherschicht (11) nicht behindert.

12. Verfahren nach Anspruch 11, umfassend einen Schritt des Aufbringens einer Dosis einer Flüssigkeit, die ein Aktivprinzip enthält, auf die Aufnahmeschicht (12).

13. Verfahren nach Anspruch 11 oder 12, bei dem eine Flüssigkeit, die ein Aktivprinzip enthält, mit dem Silikongel vor der Polymerisierung desselben gemischt wird.

14. Verfahren nach einem der Ansprüche 11 bis 13, bei dem die Aufnahmeschicht (12) aus einem Gewebe besteht, das in mindestens eine Richtung elastisch ist.

15. Verfahren nach einem der Ansprüche 11 bis 14, bei dem die Aufnahmeschicht (12) aus einem Elasthanpolyamidgewebe gewebten Typs ist.

16. Verfahren nach einem der Ansprüche 11 bis 15, bei dem die Aufnahmeschicht (12) eine Flächenmasse von einem Hundertstel oder einigen Hundertstel Gramm pro Quadratmeter und eine Dicke unter einem Millimeter aufweist.

17. Verfahren nach einem der Ansprüche 11 bis 16, bei dem die Aufnahmeschicht (12) und die Speicherschicht (11) mittels einer Klebeschicht (13) von geringer Dicke von ungefähr einem Zehntel bis zu einigen Zehntel Millimeter verbunden werden, um die Weiterleitung der Flüssigkeit, die das Aktivprinzip enthält, von der Aufnahmeschicht (12) zur Speicherschicht (11) nicht zu behindern und möglichst wenig in die Aufnahmeschicht (12) einzudringen.

18. Verfahren nach einem der Ansprüche 11 bis 17, bei dem die Speicherschicht (11) ein Polymethylsiloxan umfasst, das aus der Polymerisierung eines Silikonölgemisches stammt.

19. Verfahren nach einem der Ansprüche 11 bis 18, bei dem die Speicherschicht (11) Dimethicon oder ein Gemisch aus Dimethicon und Vinyldimethicon umfasst.

20. Verfahren nach einem der Ansprüche 11 bis 19, bei dem die erste Seite (21) der Speicherschicht (11) selbst haftend ist.

21. Verfahren nach einem der Ansprüche 11 bis 20, bei dem die Klebeschicht (13) ein Essigkleber ist.
